# EUROPÄISCHE PATENTANMELDUNG

(11) **EP 2 062 560 A2**
(43) Veröffentlichungstag der Anmeldung: **27.05.2009**
(21) Anmeldenummer: 08157477.4
(22) Anmeldetag: 03.06.2008
(51) Int. Cl.: A61K 6/02

(54) **Jaspispulver, Muschelpulver und Korallenpulver als Farbstoff in Zahnersatzmaterialien und dadurch gebildete Zahnersatzmaterialien und entsprechende Zahnprothese**

(30) Priorität: 06.06.2007 DE 102007026395
(71) Anmelder: Aichhorn, Wilfried, 79379 Mullheim/Britzinge (DE)
(72) Erfinder: Aichhorn, Wilfried, 79379 Mullheim/Britzinge (DE)
(74) Vertreter: Viering, Jentschura & Partner

(57) **Zusammenfassung**

Die vorliegende Patentanmeldung betrifft ein Verfahren zur Färbung von Zahnersatzausgangsmaterialien durch Jaspispulver, Muschelschalenpulver oder Korallenstockpulver. Die Erfindung betrifft ebenfalls ein Verfahren zur Herstellung einer Zahnersatzprothese sowie die entsprechenden Zahnersatzmaterialien und Zahnprothese.

## Beschreibung

Die vorliegende Patentanmeldung betrifft ein Verfahren zur Färbung von Zahnersatzausgangsmaterialien durch Jaspispulver, Muschelschalenpulver oder Korallenstockpulver. Die Erfindung betrifft ebenfalls ein Verfahren zur Herstellung einer Zahnersatzprothese sowie die entsprechenden Zahnersatzmaterialien und Zahnprothese.

Zahnersatzmaterialien müssen im Allgemeinen speziellen Anforderungen Stand halten. So müssen Zahnersatzmaterialien eine große Festigkeit und Haltbarkeit aufweisen, um den hohen Kaukräften bei der Speisenzerkleinerung zu widerstehen. Weiterhin sollten sich Zahnersatzmaterialien aus Gesundheitsgründen für die Verwendung im Mundraum Umgebung eignen. Wenn Zahnrestaurationen aus relativ kleinem Abstand zu erkennen sind, ist zudem die ästhetische Ausführung der Zahnprothese von großer Bedeutung. Daher ist es auf dem Gebiet der Zahnprothese üblich, Zahnersatzmaterialen entsprechend dem natürlichen menschlichen Gebiss zu färben. Als übliche Zahnersatzmaterialien werden härtbare, farblose Kunststoffe verwendet. Ästhetische Qualität bei farblosen Zahnersatzmaterialien wird erreicht, indem Materialien beigemischt werden, die zahnähnliche oder zahnfleischähnliche Farben/Farbtöne haben. Deren Färbung wird durch Beimengung von Farbmaterialien realisiert. So wird in dem deutschen Patent Nr. 802892 ein Verfahren beschrieben, bei dem pulverisierte Kunststoffe aus Polyacrylsäureverbindungen nach langsamem Erwärmen auf 40 bis 60° und langsamem Abkühlen auf 15° mit Zusätzen von Farbstoffen und farbgebenden Mineralien, wie Marmor, Quarz, Tridymit gefärbt werden. Andere gängige Färbematerialien stellt die Gruppe der Metalloxide dar. So wird üblicherweise dem Zahnersatzausgangsmaterial reines Eisenoxid, Titanoxid oder Zirkoniumoxid zur Färbung zugeführt. Bei empfindlichen Patienten führt jedoch die Zugabe von reinen Metalloxiden zu dentalen Prothesen zu nicht wünschenswerten allergischen Reaktionen. Solchen Patienten wird in Folge dessen eine verträgliche, ungefärbte, farblose Zahnprothese hergestellt. Diese farblosen Prothesen erscheinen aufgrund einer fehlenden Lichtquelle im Mund des Patienten schwarz. Die dentale Prothese hält daher den ästhetischen Anforderungen nicht mehr stand.

Es ist daher wünschenswert, ein gefärbtes Zahnersatzausgangsmaterial und eine Zahnprothese bereitzustellen, die ästhetisch annehmbar ist und darüber hinaus/zugleich keine allergische Reaktion hervorruft. Diese Aufgabe ist durch ein Verfahren gemäß der unabhängigen Patentansprüche sowie des entsprechenden Zahnersatzmaterials und Zahnprothese gelöst.

In ein solches Verfahren zur Färbung von Zahnausgangsmaterialien ist das in Kontakt bringen des Zahnausgangsmaterials mit Jaspispulver, Korallenpulver oder Muschelpulver als Färbemittel.

Folglich stellt die Erfindung ein Färbemittel zur Färbung von Zahnersatzmaterialien bereit, das zu einer wesentlich verbesserten Verträglichkeit der dentalen Prothese (Zahnprothese) beiträgt.

Überraschenderweise wurde in der Erfindung entdeckt, dass sich Jaspis, Koralle und Muschelschale für die Färbung von Zahnersatzmaterial sehr gut eignen und zugleich keinerlei allergische Reaktion hervorrufen. Diese Materialien sind kommerziell sehr gut erhältlich und eröffnen somit einen eleganten Zugang zu einer neuen, vorteilhaften und kostengünstigen hypoallergenen Zahnprothese. Der Begriff Jaspis wird in der vorliegenden Erfindung in seiner üblichen Bedeutung verwendet, das heißt als nicht-transparente Abart von Quarz (siehe Holleman, Wiberg; Lehrbuch der anoganischen Chemie, 91 - 100ste Auflage, Seiten 750 - 751). Illustrative Beispiele von einsetzbaren Jaspisarten sind Achatjaspis, Ägyptische Jaspis, Bandjaspis, Kellerwaldachat (weißgeäderten roten Jaspis), Mookait, Nunkirchner Jaspis, Porzellanjaspis und Zebrajaspis.

Für die Verwendung in der vorliegenden Erfindung werden Jaspis, Koralle und Muschelschale üblicherweise in Pulverform eingesetzt. Eine solche Pulverisierung kann beispielsweise durch Diamantschleifer oder Kugelmühle erhalten werden. Die Pulverpartikel können jede für die Einfärbung geeignete Größe aufweisen. Beispielsweise kann die durchschnittliche Partikelgröße 0,1 bis 50 µm, einschließlich zum Beispiel 20 oder 40 µm betragen. Falls gewünscht kann auch eine größere Partikelgröße verwendet werden.

In einer Ausführungsform des Verfahrens zur Färbung des Zahnersatzausgangsmaterials wird das Zahnersatzausgangsmaterial in Granulat- oder Pulverform vorgelegt und das Färbemittel wird in Pulverform im Überschuss bezogen auf die freie Bindungskapazität des Zahnersatzausgangsmaterial hinzugegeben, derart dass bei Vermengung das Färbemittelpulver durch Adhäsion die verfügbaren Bindungsstellen der Oberfläche des Zahnersatzmaterials absättigt.

Überschüssiges Färbemittelpulver kann in dieser Ausführungsform von dem Gemisch des Zahnersatzmaterials, dessen Bindungsstellen an der Oberfläche von Färbemittelpulver durch Adhäsionskräfte abgesättigt sind, durch gängige Methoden abgetrennt werden. Beispielsweise kann dies erfolgen, indem die Mischung auf ein schräg gelegtes Leinentuch gegeben wird, wobei während des Hinabrollens das freie Färbemittelpulver an das Leinentuch abgegeben wird. Erhalten wird dadurch ein Zahnersatzausgangsmaterial, dessen Granulat/Pulver gleichmäßig mit Färbemittelpulver bedeckt ist. In dieser Ausführungsform ist es daher nicht notwendig, exakte Mengenverhältnisse einzuhalten, es genügt lediglich, dass das Färbemittelpulver im Überschuss vorgelegt werden kann. Dies ermöglicht eine optimale Färbung des Zahnersatzmaterials. Allerdings ist es auch möglich, einen Unterschuß an Färbematerial zu nehmen, falls dadurch ein gewünschter Farbton erhalten werden kann. Allerdings ist es ebenfalls möglich bestimmte Mengenverhältnisse in dem erfindungsgemäßen Verfahren einzusetzen. Dies gilt insbesondere, falls das Zahnersatzausgangsmaterial in flüssiger Form vorliegt

Das Verfahren zur erfindungsgemäßen Färbung des Zahnersatzmaterials umfasst in einer weiteren Ausführungsform
(a) ein härtbares Harz (beziehungsweise einen härtbaren Kunststoff) in flüssiger oder hochviskoser Form/pastenförmiger Form vorzulegen und
(b) ein Färbemittelpulver vorzulegen, wobei das Färbemittelpulver (gegebenenfalls in einer zuvor bestimmten Menge) dem in flüssiger oder pastenförmiger Form vorliegenden Zahnersatzausgangsmaterial zugegeben wird.

Die Menge an Färbemittelpulver kann abhängen von dem Grad der gewünschten Färbung und der Intensität des Färbemittelpulvers und kann vom Fachmann leicht bestimmt werden. Beispielsweise kann das Färbemittelpulver dem in flüssiger Form vorgegebenen Zahnersatzausgangsmaterial in einer Menge von circa 0,1 g bis 40 g bezogen auf 1 l Flüssigkeit zugegeben werden. In einer weiteren Ausführungsform können circa auch 0,5 g bis 20 g Färbemittelpulver zu dem Zahnersatzmaterial bezogen auf 1 1 Flüssigkeit zugegeben werden. Anschaulich ausgedrückt kann die Menge des Färbepulvers 1 Esslöffel pro 1 1 Flüssigkeit betragen. Dieselben Mischungsverhältnisse können ebenfalls bei dem in Pulverform oder pastenförmiger Form vorliegenden Zahnersatzausgangsmaterial verwendet werden. Das Färbematerial wird üblicherweise durch Schütteln oder Rühren gleichmäßig in der Flüssigkeit verteilt. Das Farbmaterial im Gemisch mit pastenförmigem Zahnersatzausgangsmaterial wird üblicherweise durch dünnes Auswalzen mittels einer Walze gleichmäßig verteilt.

Die wie vorliegend beschriebene Mischung aus Zahnersatzausgangsmaterial und Färbemittel wird bei Vorliegen eines Zahnersatzmaterials in Granulatform erhitzt und dann mittels konventioneller Formgebungsverfahren wie beispielsweise Injektionsverfahren geformt. Falls die Mischung in flüssiger/dispergierter Form vorliegt, kann diese zur Aushärtung ebenfalls in einer Form erhitzt werden. Diese Formgebung erfolgt ebenfalls in einer dem Fachmann geläufigen Weise.

In einer Ausführungsform werden die Färbemittel Jaspispulver sowie Korallenpulver aufgrund ihrer rötlichen Farbe zur Färbung des Teils der Zahnprothese verwendet, die das künstliche Zahnfleisch darstellen. Das Muschelschalenpulver kann dagegen aufgrund seiner weißen Farbe zur Färbung der Zähne der Zahnprothese verwendet werden. Farbige Muschelschalen können ebenfalls zur Färbung der Zahnprothese verwendet werden, die das künstliche Zahnfleisch darstellen.

Das fertige Gemisch aus Zahnersatzausgangsmaterial und Färbepulver kann zur weiteren Verarbeitung problemlos gelagert werden. Im Falle einer flüssigen Mischung kann es notwendig sein, den in der Flüssigkeit befindlichen Farbstoff erneut aufzurühren.

In einer weiteren Ausführungsform der Erfindung wird das gefärbte Zahnersatzmaterial (das heißt die Mischung aus Zahnersatzmaterial und Färbemittel) zur Bildung einer Zahnprothese verwendet wird.

Harze, das heißt Zahnersatzausgangsmaterialien auf Kunststoffbasis, die für die erfindungsmäßigen Materialien nützlich sind, sind im Allgemeinen wärmehärtbare (thermoplastische) Harze, die durch Erwärmen Polymernetzwerke bilden. Geeignete Harze umfassen Acetylatharze, Methacetylatharze, Epoxidharze, Vinylharze und Mischungen davon. Das härtbare Harz wird vorzugsweise aus einem oder mehr matrixbildenden Oligomer, Monomer oder Polymer oder Gemischen davon hergestellt.

In einer weiteren Ausführungsform umfassen geeignete polymerisierbare Harze härtbare organische Harze mit hinreichender Festigkeit, hydrolytischer Stabilität und Ungiftigkeit, die zur Verwendung in der oralen Umgebung geeignet sind. Beispiele deratiger Harze umfassen Acrylat-, Methacrylat-, Urethan-, Carbamoylisocyanurat-, Epoxidharze und Mischungen und Derivate davon. Derartige Harze werden in den US-Patentschriften Nr. 3,066,112, 3,539533, 3,629,187, 3,709,866, 3,751,399, 3,766,132, 3,860,556, 4,002,669, 4,115,346, 4,259,117, 4,292,029, 4,308,190, 4,327,014, 4,379,695, 4,387,240 und 4,40,150 offenbart.

Eine weitere Klasse härtbarer Harze (Zahnersatzausgangsmaterialien) sind Materialien mit radikalisch aktiven funktionellen Gruppen und umfassen Mononomere, Oligomere und Polymere mit einer oder mehrerer ethylenisch ungesättigten Gruppen. Alternativ kann das härtbare Harz die Klasse der Harze mit kationisch aktiven funktionellen Gruppen umfassen. In einer weiteren Alternative kann eine Mischung härtbarer Harze, die sowohl kationisch aushärtbare als auch radikalisch aushärtbare Harze umfasst, verwendet werden.

In der Klasse mit härtbaren Harzen mit radikalische aktiven funktionellen Gruppen enthalten geeignete Materialien zur Verwendung in der Erfindung mindestens eine ethylenisch ungesättigte Bindung und sind in der Lage, sich einer Additionspolymerisation zu unterziehen. Derartige radikalisch polymerisierbare Materialien umfassen Mono-, Di-, oder Polyacrylate und -methacrylate, wie Methylacrylat, Methylmethacrylat, Ethylacrylat, Isopropylmethacrylat, n-Hexylacrylat, Stearylacrylat, Alylacrylat, Glycerindiacrylat, Glycerintriacrylat, Ethylenglycoldiacrylat, Diethylenglycoldiacrylat, Triethylenglycodimethacrylat, 1,3-Propandiolacrylat, 1,3-Propandildi(meth)acrylat, Trimethylolprpantriacrylat, 1,2,4-Butantrioltrimethacrylat, 1,4-Cyclohexandioldiacrylat, Pentaerythrittriacrylat, Pentaerythrittetramethacrylat, Sorbitolhexaacrylat, das Diglycidylmethacrylat von Bisphenol A ("Bis-GMA"), Bis[1-(2-acryloxy)]-p-ethoxyphenyldimethylmethan, Bis[1-(3-acryloxy-2hydroxy)]-p-p-ropoxyphenyldimethylmethan und Trishydroxyethylisocyanurattrimethacrylat; die Bisacrylate und Bismethacrylate von Polyethylenglycolen mit einem Molekulargewicht von 200 bis 500, copoymerisierbare Mischungen von Acrylatmonomeren, wie in US-Patentschrifr Nr. 4,652,274 und Acrylatoligomere, wie die der US-Patentschrift Nr. 4,642,126; und Vinylverbindungen, wie Styren, Dialylphtalat, Divinylsuccinat, Divinyladipat und Divinylphthalat. Mischungen von zwei oder mehr dieser radikalisch polymerisierbaren Materialien können als Zahnersatzmaterial gemäß der Erfindung verwendet werden.

Beispiele für besonders gut geeignete Zahnersatzausgangsmaterialien mit geringer geringer Allergenizität schließen Polyamid, Naturkautschuk, Polyarylether, Polyaryletherketon, Polyethylen, Polypropylen, Copolimer, Polycarbonat, Polyester und Polybutylenteraphthalat ein.

Für eine Radikalpolymerisation (Härtung) kann ein Initiatorsystem gewählt werden, die die Polymerisation mittels Strahlung, Wärme oder chemischer Redox-/Selbstaushärtungsreaktion initiieren.

Das erfindungsgemäße gefärbte Zahnersatzmaterial kann in allen beliebigen Dentalanwendungen verwendet werden. Derartige Anwendungen umfassen Dentalklebstoffe, künstliche Zahnkronen, anteriore und posteriore Füllungen, Beschichtungszusammensetzungen, Formlinge, orthodontische Vorrichtungen, Füllungen, Zahnersatz, Versiegelungsmittel und dentale Vollprothesen. Das gefärbte Zahnersatzmaterial kann in dem Mund platziert und dort an Ort und Stelle ausgehärtet oder gehärtet werden. Alternativ kann es außerhalb des Mundes zu einer Prothese gefertigt und anschließend an die Stelle im Mund geklebt werden.

Wie hierin verwendet, ist die "ästhetische Qualität" eines Materials, insbesondere eines Zahnersatzmaterial zwar eine subjektive Charakteristik trotzdem eine im Allgemeinen gut verstandenen Eigenschaft in der Zahntechnik. Sie kann, falls gewünscht, durch Messung der Opazität quantifiziert werden.

"Härtbar" beschreibt in der vorliegenden Erfindung ein Zahnersatzausgangsmaterial, das ausgehärtet oder verfestigt werden kann, beispielsweise durch Erwärmen, um das Lösemittel zu entfernen, Erwärmen, um Polymerisation zu verursachen (thermoplastisch), chemisches Vernetzen (chemoplastisch), strahlungsinduzierte Polymerisation oder Vernetzung;

Eine weitere Klasse härtbarer Harze sind Materialien mit kationisch aktiven funktionellen Gruppen. Materialien mit kationisch aktiven funktionellen Gruppen umfassen kationisch polymerisierbare Epoxidharze, Vinylether, Oxetane, Spiroothocarbonate, Spiroorthoester und desgleichen.

Die Erfindung wird durch die nachfolgenden Ausgangsbeispiele näher erläutert:
1. Jaspisstein wird mittels eines Diamentenschleifers zu Jaspispulver verarbeitet, dem Zahnersatzausgangsmaterial beigemischt und wie folgt eingesetzt:
   Das Gemisch aus Jaspispulver und thermoplastischem Zahnersatzausgangsmaterial in Granulat- (ähnlich Milchreiskörnern) oder Pulverform wird in einer zylindrischen Kartusche, bevorzugt aus Aluminium, gegeben und bis zum Schmelzpunkt des Zahnersatzausgangsmaterials erhitzt. Üblicherweise werden thermoplastische Zahnersatzausgangsmaterialien bis zu 400 °C erhitzt. Mittels eines pressluftbetriebenen Kolbens wird die Kartusche zusammengepresst und das erhitzte, gefärbte Zahnersatzmaterial zur Aushärtung in eine Hohlform gespritzt. Ist die Aushärtung erreicht, wird die Hohlform mittels eines Presslufthammers stückweise entfernt. Die entstandene Zahnprothese wird geschliffen, geglättet und poliert.
2. Heißpolymerisate (beispielsweise Methyl-methacrylat, Vinyl und Polyurethandimethacrylat), bei denen das Jaspispulver nach Gewicht und abhängig von der gewünschten Färbung und Farbintensität beigemischt wird, sind in Pulverform oder in Pastenform kommerziell erhältlich. Das Heißpolymerisat in Pulverform wird mit einer Flüssigkeit gemischt und es entsteht eine Paste. Diese Paste oder auch das kommerziell erwerbliche pastenförmige Zahnersatzausgangsmaterial wird in eine Walze gegeben und das Jaspispulver, abhängig von der gewünschten Färbung und Farbintensität während des Walzenvorgangs beigemischt. Das dünne Auswalzen der Paste bewirkt eine gleichmäßige Verteilung des Jaspispulvers. Die gefärbte Paste wird in eine Hohlform eingebracht und diese geschlossen. Mit Druckausübung auf die Hohlform dringt die überschüssige, gefärbte Paste aus der Hohlform. Für die Überführung der Paste in die Hohlform kann eine Injektionsmaschine verwendet werde, die jedoch langsamer und nur mit Polymerisationswärme von beispielsweise bis zu 100 °C arbeitet. Die Aushärtung des gefärbten Zahnersatzausgangsmaterials wird mittels Abkühlung erreicht. Ist die Aushärtung erreicht, wird die Hohlform mittels eines Presslufthammers stückweise entfernt. Die entstandene Zahnprothese wird geschliffen, geglättet und poliert.

## Patentansprüche

1. Verfahren zur Färbung eines Zahnersatzausgangsmaterials, wobei das Zahnersatzausgangsmaterial mit Jaspispulver, Korallenpulver oder Muschelschalenpulver als Färbemittel in Kontakt gebracht wird.

2. Verfahren gemäß Anspruch 1, wobei das Zahnersatzausgangsmaterial lichthärtenden, chemoplastischen oder thermoplastische Kunststoff oder eine Mischung davon enthält.

3. Verfahren gemäß Anspruch 1 oder 2, wobei das Zahnersatzausgangsmaterial in Granulatform vorgelegt wird.

4. Verfahren gemäß Anspruch 4, wobei das färbemittel in Pulverform im Überschuss bezogen auf die freie Bindungskapazität des Zahnersatzausgangsmaterial hinzugegeben wird, so dass bei Vermengung das Färbemittelpulver durch Adhäsion die verfügbaren Bindungsstellen der Oberfläche des Zahnersatzmaterials absättigt.

5. Verfahren gemäß Anspruch 1 oder 2, wobei das Färbemittelpulver dem in flüssiger oder pastenförmiger Form vorliegenden Zahnersatzausgangsmaterial hinzugegeben wird.

6. Verfahren gemäß Anspruch 5, wobei das Färbemittelpulver zu dem Zahnersatzmaterial in einer Menge von 0,1 g bis 40g bezogen auf 1 1 Flüssigkeit eingesetzt wird.

7. Verfahren gemäß Ansprüchen 1 bis 6, wobei das gefärbte Zahnersatzmaterial zur Bildung einer Zahnprothese verwendet wird.

8. Zahnersatzmaterial erhältlich durch ein Verfahren gemäß einem der Ansprüche 1 bis 6.

9. Verfahren zur Herstellung einer Zahnprothese, wobei ein Zahnersatzmaterial gemäß Ansprüchen 1 bis 7 in einer entsprechenden Form ausgehärtet wird.

10. Verfahren gemäß dem Anspruch 9, wobei das gefärbte Zahnersatzmaterial zur Nachbildung von künstlichen Zähnen verwendet wird.

11. Verfahren gemäß dem Anspruch 9, wobei das gefärbte Zahnersatzmaterial zur Nachbildung von künstlichem Zahnfleisch verwendet wird.

12. Verwendung von Jaspispulver, Korallenpulver oder Muschelschalenpulver als Färbematerial für Zahnersatzmaterialien.

13. Zahnprothese erhältlich durch ein Verfahren gemäß einem der Ansprüche 9 bis 11.
